# EUROPEAN PATENT APPLICATION

(11) **EP 3 290 984 A1**
(43) Date of publication of application: **07.03.2018**
(21) Application number: 16786240.8
(22) Date of filing: 22.03.2016
(51) Int. Cl.: G02B 21/00, G01N 21/27

(54) **MICROSCOPE DEVICE**

(30) Priority: 30.04.2015 JP 2015093072
(71) Applicant: Sumitomo Electric Industries, Ltd., Osaka-shi, Osaka 541-0041 (JP)
(72) Inventor: SUGANUMA Hiroshi, Yokohama-shi Kanagawa 244-8588 (JP); OKUNO Takuya, Yokohama-shi Kanagawa 2448588 (JP); OKINA Yuna, Yokohama-shi Kanagawa 2448588 (JP); MOTOMURA Asako, Yokohama-shi Kanagawa 2448588 (JP); SUGIYAMA Yoko, Yokohama-shi Kanagawa 2448588 (JP)
(74) Representative: Hoffmann Eitle
(86) International application number: PCT/JP2016/059005
(87) International publication number: WO 2016/174963

(57) **Abstract**

Provided is a microscope device which includes: (1) a light source (10) that outputs illumination light in a wavelength band including a near-infrared region; (2) an illumination optical system (30) that irradiates an observation target (90) with the illumination light output from the light source; (3) an image formation optical system (40) that includes a spectroscopic unit which is configured to receive and disperse transmitted or scattered light produced at the observation target by irradiating the observation target with the illumination light, and that forms an image on the basis of the dispersed transmitted or scattered light; and (4) an image capturing unit (50A) that acquires the image formed by the image formation optical system. An illumination-light-irradiated area of the observation target is larger than an area of the field of view of the image formation optical system (40), and is less than or equal to ten times the area of the field of view.

## Description

### Technical Field

The present invention relates to a microscope device.

### Background Art

When an observation target is observed, the observation target is irradiated with illumination light output from a light source by an illumination optical system, and an image of the observation target is formed by an image formation optical system including an objective lens that inputs light (transmitted light or diffuse reflected light) produced at the observation target, and is acquired by an image capturing unit, so that the observation can be made on the basis of the acquired image.

Especially, when the observation target is living tissue (a cell), the cell is transparent in a visible region, and thus illumination light of a near-infrared region can be favorably used (see Patent Literature 1). A hyperspectral image having spectral information of light occurring at each position of the observation target is acquired, so that the observation target can be analyzed on the basis of the hyperspectral image.

The invention disclosed in Patent Literature 1 irradiates the living tissue with the illumination light of the near-infrared region, receives and analyzes the transmitted light or the reflected light from the living tissue, and thereby obtains distribution of chemical substances in the living tissue.

An invention disclosed in Patent Literature 2 irradiates an observation target with a given band of illumination light, selects light of a specific wavelength from transmitted light or reflected light from the observation target using a bandpass filter, and makes a spectroscopic analysis on the basis of the selected light having a specific wavelength.

### Citation List

### Patent Literature

[Patent Literature 1] Japanese Unexamined Patent Publication No. 2011-237178
[Patent Literature 2] Japanese Unexamined Patent Publication No. 2014-95594

### Summary of Invention

### Technical Problem

The invention disclosed in Patent Literature 1 irradiates the living tissue with illumination light having any one of specific wavelengths of 770 nm, 808 nm, and 854 nm in the near-infrared region in order to obtain the distribution of the specific substances in the living tissue. This invention can acquire neither spectral information over a given band of the light produced at the observation target nor a hyperspectral image.

In contrast, the invention disclosed in Patent Literature 2 can acquire spectral information that is substantially continuous over a given band of the light produced at the observation target. However, in this invention, the observation target is irradiated with illumination light in a given band, whereas only the light of specific wavelengths transmitted by the bandpass filter from the transmitted light or the reflected light from the observation target is detected. Thus, the observation target is further irradiated with light having undetected wavelengths, and therefore damage to the living tissue that is the observation target is great.

The present invention was made to overcome the above problems, and an object thereof is to provide a microscope device capable of acquiring an image having spectral information that is substantially continuous over a given band while suppressing damage to an observation target.

### Solution to Problem

A microscope device of the present invention includes: (1) a light source configured to output illumination light in a wavelength band including a near-infrared region; (2) an illumination optical system configured to irradiate an observation target with the illumination light output from the light source; (3) an image formation optical system including a spectroscopic unit which is configured to receive and disperse transmitted or scattered light produced at the observation target by irradiating the observation target with the illumination light, and configured to form an image on the basis of the dispersed transmitted or scattered light; and (4) an image capturing unit configured to acquire the image formed by the image formation optical system. An illumination-light-irradiated area of the observation target is larger than an area of the field of view of the image formation optical system, and is less than or equal to ten times the area of the field of view.

### Advantageous Effects of Invention

According to the present invention, an image having spectral information that is substantially continuous over a given band can be acquired while suppressing damage to an observation target.

### Brief Description of Drawings

Fig. 1 is a diagram illustrating a configuration of a microscope device 1A of a first embodiment.
Fig. 2 is a diagram illustrating a configuration of a microscope device 1B of a second embodiment.

### Description of Embodiments

A microscope device of the present invention includes: (1) a light source configured to output illumination light in a wavelength band including a near-infrared region; (2) an illumination optical system configured to irradiate an observation target with the illumination light output from the light source; (3) an image formation optical system including a spectroscopic unit which is configured to receive and disperse transmitted or scattered light produced at the observation target by irradiating the observation target with the illumination light, and configured to form an image on the basis of the dispersed transmitted or scattered light; and (4) an image capturing unit configured to acquire the image formed by the image formation optical system.

Alternatively, the microscope device of the present invention includes: (1) a light source configured to output illumination light in a wavelength band including a near-infirared region; (2) a spectroscope configured to disperse the illumination light output from the light source and to output the dispersed illumination light at each wavelength; (3) an illumination optical system configured to irradiate an observation target with the illumination light of each wavelength output from the spectroscope; (4) an image formation optical system configured to receive transmitted or scattered light produced at the observation target by irradiating the observation target with the illumination light and to form an image on the basis of the input transmitted or scattered light; and (5) an image capturing unit configured to acquire the image formed by the image formation optical system.

In the present invention, an illumination-light-irradiated area of the observation target is larger than an area of the field of view of the image formation optical system, and is less than or equal to ten times the area of the field of view. It is more preferable if the area of the observation target is less than or equal to twice the area of the field of view.

In the present invention, the image formation optical system is preferably a double-sided telecentric optical system. Further, the illumination optical system irradiates the observation target with the illumination light as collimated light. Further, the illumination optical system preferably irradiates the observation target with the illumination light as converging light. Further, the microscope device preferably further includes a filter configured to interrupt light in a wavelength band of all or some wavelengths other than wavelengths used for measurement out of the illumination light output from the light source. Further, the illumination optical system preferably further includes a removable filter for adjusting an amount of light. Further, the image capturing unit is preferably configured to acquire a hyperspectral image on the basis of transmitted light or diffuse reflected light produced at the observation target.

Hereinafter, a mode for carrying out the present invention will be described in detail with reference to the attached drawings. In the description of the drawings, the same reference signs are given to the same elements, and duplicate description will be omitted. The present invention is not limited to these examples. The scope of the present invention is defined by the claims, and is intended to include all the modifications and alternations within the meanings and range equivalent to the claims.

### (First embodiment)

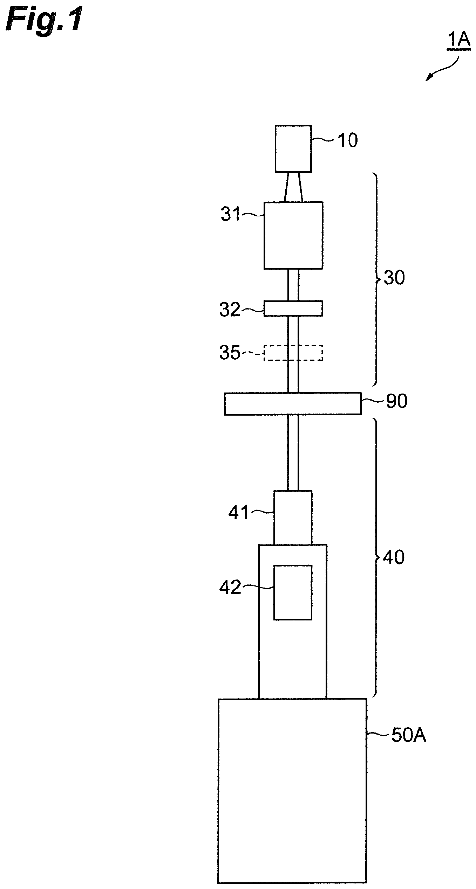

Fig. 1 is a diagram illustrating a configuration of a microscope device 1A of a first embodiment. A microscope device 1A includes a light source 10, an illumination optical system 30, an image formation optical system 40, and an image capturing unit 50A, and acquires a hyperspectral image of an observation target 90.

The light source 10 outputs illumination light in a wavelength band including a near-infrared region (e.g., 1000 to 2500 nm). The light source 10 preferably has output intensity in a wide band. It is preferable that the light source 10 be for instance a halogen lamp or a xenon lamp.

The illumination optical system 30 irradiates the observation target 90 with the illumination light output from the light source 10. The illumination optical system 30 includes a collimator lens 31 and a filter 32. The collimator lens 31 collimates the illumination light that is emanated and output from the light source 10. The filter 32 inputs the illumination light collimated by the collimator lens 31, selectively transmits light in a near-infrared region out of the illumination light, and interrupts light of both or one of an ultraviolet region (e.g., 170 to 375 nm) and an infrared region (e.g., 2400 to 5000 nm). The filter 32 may be configured to interrupt light in a wavelength band of all or some wavelengths other than wavelengths used for measurement out of the illumination light. That is, the filter 32 may interrupt light in a visible region (e.g., 375 nm to 780 nm). When only light in a specific wavelength band out of the light in the near-infrared region is used for measurement, the filter may be configured to interrupt a region other than the near-infrared region excluding the wavelengths used for measurement.

The observation target 90 is for instance living tissue (a cell), and is contained in a container along with a culture solution, and is placed on a stage. The stage can be displaced in two directions perpendicular to an optical axis of an objective lens 41, and can also be displaced in a direction parallel to the optical axis of the objective lens 41.

The image formation optical system 40 includes the objective lens 41 and a spectroscope 42, inputs light (transmitted light) produced at the observation target 90 by irradiating the observation target 90 with the illumination light into the objective lens 41, and forms an image on the basis of the input light. The spectroscope 42 includes a spectroscopic element such as a prism or a grism, a grating, a Fourier spectroscope, etc., and disperses the input light to output it at each wavelength. Instead of the spectroscope 42, a wavelength selective filter may be used as means for inputting and dispersing the light produced at the observation target.

The image capturing unit 50A acquires the image formed by the image formation optical system 40. An image capturing device includes a two-dimensional sensor that is formed of, for instance, InGaAs or HgCdTe and is sensitive to the near-infrared region, and receives light of each wavelength output from the spectroscope to acquire an image.

In the related art, in comparison with the case in which the hyperspectral image in the visible region is acquired, in the case in which the hyperspectral image in the near-infrared region is acquired, there was a need to irradiate the observation target 90 with illumination light having stronger intensity in order to obtain effective spectral information. The light output from the light source 10 is generally diverging light. When the illumination light that is the diverging light is applied to the observation target 90, the light in the ultraviolet region or the infrared region is also applied along with the light in the near-infrared region according to power or irradiation time of the illumination light, and thus damage caused to the living tissue that is the observation target 90 is great.

Therefore, in the present embodiment, the area of the observation target 90 which is irradiated with the illumination light is larger than an area of a field of view of the image formation optical system 40 at a position of the observation target 90, and is smaller than or equal to ten times the area of the field of view The area of the field of view is decided by an area of an image forming plane of the image capturing unit 50A and a magnification of the objective lens 41, and becomes 1/100 times the area of the image forming plane, for instance, when the objective lens of 10× magnification is used. With this configuration, damage to a region of the observation target 90 which excludes an observation region can be suppressed to acquire the hyperspectral image.

In the present embodiment, the illumination optical system 30 irradiates the observation target 90 with the illumination light that is formed into collimated light by the collimator lens 31. Thereby, generation of scattered light or diffracted light from the observation target 90 can be suppressed, the transmitted light from the observation target 90 can be detected with high sensitivity, and irregularity of the hyperspectral image can be prevented.

In the present embodiment, the illumination optical system 30 interrupts the light in the wavelength band of all or some wavelengths other than the wavelengths used for measurement out of the illumination light using the filter 32, and selectively transmits the remaining light to irradiate the observation target 90. Thereby, light of wavelengths that are not used for observation is inhibited from being applied to the observation target 90, and the damage to the observation target 90 can be suppressed.

In the present embodiment, the image formation optical system 40 is preferably a double-sided telecentric optical system. With this configuration, stray light in forming the image can be reduced, dependence on an incidence angle of the light on the spectroscope of the image capturing unit 50A can be removed, and a variation in in-plane wavelength is hardly caused.

In the present embodiment, the illumination optical system 30 may include a removable neutral density (ND) filter 35 for adjusting an amount of light. As illustrated in Fig. 1, the ND filter 35 can be provided in a rear stage of the filter 32. The ND filter 35 can be used when background measurement of the illumination light is performed. When evaluation of the observation target 90 is carried out, the evaluation is generally performed after the background measurement is performed to capture a hyperspectral image concerning the illumination light that does not pass through the observation target 90, and a difference in the spectral information between the captured hyperspectral image and a hyperspectral image obtained by capturing an image of the transmitted light from the observation target 90 is found. Here, when an amount of the transmitted light from the observation target 90 is sufficiently smaller than an amount of background light that does not pass through the observation target 90, it is considered that a component derived from a difference in the amount of light is included in a result of calculating the difference in the spectral information. In contrast, the removable ND filter 35 is configured to be used to adjust an amount of light incident on the image capturing unit 50A when the background light that does not pass through the observation target 90 is measured, and thereby calculation accuracy of the difference in the spectral information based on the background measurement can be enhanced.

### (Second embodiment)

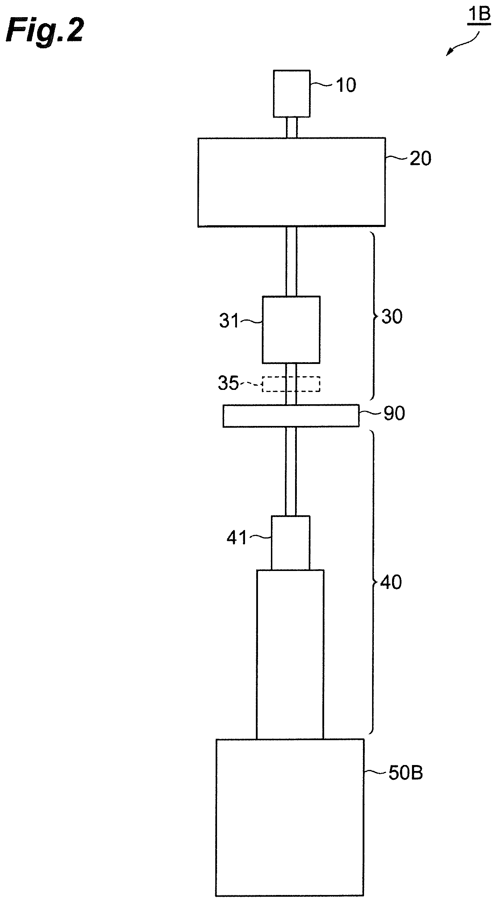

Fig. 2 is a diagram illustrating a configuration of a microscope device 1B of a second embodiment. A microscope device 1B includes a light source 10, a spectroscope 20, an illumination optical system 30, an image formation optical system 40, and an image capturing unit 50B, and acquires a hyperspectral image of an observation target 90.

In comparison with the configuration of the microscope device 1A of the first embodiment illustrated in Fig. 1, the microscope device 1B of the second embodiment illustrated in Fig. 2 is different in that the spectroscope 20 is provided at a side of incidence of the observation target 90, in that the illumination optical system 30 may not include a filter 32, and in that an image capturing unit 50B is provided instead of the image capturing unit 50A.

The spectroscope 20 includes a spectroscopic element such as a prism or a grism, disperses illumination light output from the light source 10, and sequentially outputs the dispersed illumination light at each wavelength. If the light output by the spectroscope 20 is restricted within a near-infrared region, the illumination optical system 30 may not include the filter 32. The spectroscope 20 may include a spectroscopic element such as a grating, a Fourier spectroscope, or the like. Instead of the spectroscope 20, a wavelength selective filter may be used as means for dispersing light applied to an observation target. The illumination optical system 30 irradiates the observation target 90 with the illumination light of each wavelength which is sequentially output from the spectroscope 20. The illumination optical system 30 includes a collimator lens 31 that collimates the illumination light output from the spectroscope 20.

The image capturing unit 50B acquires an image formed by the image formation optical system 40. The image capturing unit 50B does not include the spectroscope. The image capturing unit 50B includes a two-dimensional sensor that is formed of, for instance, InGaAs or HgCdTe and is sensitive to the near-infrared region as an image capturing device, and receives light of each wavelength that has arrived from the image formation optical system 40 to acquire an image.

Even in the second embodiment, an area of the observation target 90 which is irradiated with the illumination light is larger than an area of a field of view of the image formation optical system 40 at a position of the observation target 90, and is set to be smaller than or equal to ten times the area of the field of view. The illumination optical system 30 irradiates the observation target 90 with the illumination light that is formed into collimated light by the collimator lens 31. The image formation optical system 40 is preferably a double-sided telecentric optical system. The microscope device 1B of the second embodiment also exerts the same effect as the microscope device 1 A of the first embodiment.

In the second embodiment, the light output by the spectroscope 20 can be restricted within the near-infrared region. Thus, in view of this, light having wavelengths that are not used for observation can be inhibited from being applied to the observation target 90, and damage to the observation target 90 can be suppressed.

Even in the second embodiment, the illumination optical system 30 may include a removable ND filter 35 for adjusting an amount of light. As illustrated in Fig. 2, the ND filter 35 can be provided in a rear stage of the collimator lens 31. Like the first embodiment, an amount of light incident on the image capturing unit 50A when background light that does not pass through the observation target 90 is measured is configured to be adjusted using the removable ND filter 35, and thereby calculation accuracy of a difference in spectral information based on background measurement can be enhanced.

### (Modification)

The present invention is not limited to the above embodiments, and can be modified in various ways. For example, the image capturing unit acquires the hyperspectral image on the basis of the transmitted light produced at the observation target in the above embodiments, but it may acquire the hyperspectral image on the basis of the diffuse reflected light produced at the observation target.

In the illumination optical system 30 that irradiates the observation target with the illumination light output from the light source, the configuration in which the illumination light formed into the collimated light by the collimator lens 31 is applied to the observation target 90 has been described, but the collimator lens 31 can be changed into a condenser lens. That is, in each of the microscope device 1A of the first embodiment illustrated in Fig. 1 and the microscope device 1B of the second embodiment illustrated in Fig. 2, the condenser lens may be arranged instead of the collimator lens 31. In addition, the condenser lens may be arranged in a rear stage of the collimator lens 31. When the illumination optical system 30 includes the condenser lens, converging light condensed by the condenser lens is applied to the observation target 90.

For example, when the objective lens is set to 2.5× magnification, if the illumination optical system 30 adopts a condenser lens having 5× magnification, a beam diameter of the illumination light applied to the observation target 90b can be controlled to be small, and an area irradiated with the illumination light can be set to be less than or equal to twice the area of the field of view. When the objective lens is set to 10× magnification, if the illumination optical system 30 adopts a condenser lens having 10× magnification, a beam diameter of the illumination light applied to the observation target 90b can be controlled to be small, and an area irradiated with the illumination light can be set to be less than or equal to twice the area of the field of view. In this way, the converging light condensed by the condenser lens is configured to be applied to the observation target 90. Thereby, illumination light having a higher light condensing rate can be applied to a region of the field of view on the observation target 90 required to acquire the hyperspectral image.

The illumination optical system that irradiates the observation target with the illumination light output from the light source may include an optical fiber that guides the illumination light output from the light source. In this case, since an emitting end of the optical fiber can be regarded as a point light source, illumination light emitted from the emitting end of the optical fiber is collimated by the collimator lens, and thereby a spot diameter of the illumination light applied to the observation target 90 can be set to be smaller.

### Reference Signs List

- 1A, 1B: Microscope device
- 10: Light source
- 20: Spectroscope
- 30: Illumination optical system
- 31: Collimator lens
- 32: Filter
- 35: ND filter
- 40: Image formation optical system
- 41: Objective lens
- 42: Spectroscope
- 50A, 50B: Image capturing unit
- 90: Observation target

## Claims

1. A microscope device comprising:
a light source configured to output illumination light in a wavelength band including a near-infrared region;
an illumination optical system configured to irradiate an observation target with the illumination light output from the light source;
an image formation optical system including a spectroscopic unit which is configured to receive and disperse transmitted or scattered light produced at the observation target by irradiating the observation target with the illumination light, and configured to form an image on the basis of the dispersed transmitted or scattered light; and
an image capturing unit configured to acquire the image formed by the image formation optical system,
wherein an illumination-light-irradiated area of the observation target is larger than an area of a field of view of the image formation optical system, and is less than or equal to ten times the area of the field of view.

2. A microscope device comprising:
a light source configured to output illumination light in a wavelength band including a near-infrared region;
a spectroscope configured to disperse the illumination light output from the light source and to output the dispersed illumination light at each wavelength;
an illumination optical system configured to irradiate an observation target with the illumination light of each wavelength output from the spectroscope;
an image formation optical system configured to receive transmitted or scattered light produced at the observation target by irradiating the observation target with the illumination light and to form an image on the basis of the input transmitted or scattered light; and
an image capturing unit configured to acquire the image formed by the image formation optical system,
wherein an illumination-light-irradiated area of the observation target is larger than an area of a field of view of the image formation optical system, and is less than or equal to ten times the area of the field of view.

3. The microscope device according to claim 1 or 2, wherein the image formation optical system is a double-sided telecentric optical system.

4. The microscope device according to any one of claims 1 to 3, wherein the illumination optical system irradiates the observation target with the illumination light as collimated light.

5. The microscope device according to any one of claims 1 to 3, wherein the illumination optical system irradiates the observation target with the illumination light as converging light.

6. The microscope device according to any one of claims 1 to 5, further comprising a filter configured to interrupt light in a wavelength band of all or some wavelengths other than wavelengths used for measurement out of the illumination light output from the light source.

7. The microscope device according to any one of claims 1 to 6, wherein the illumination optical system further includes a removable filter for adjusting an amount of light.

8. The microscope device according to any one of claims 1 to 7, wherein the image capturing unit is configured to acquire a hyperspectral image on the basis of transmitted light or diffuse reflected light produced at the observation target.
